# FASCICULE DE BREVET EUROPEEN

(11) **EP 3 539 470 B1**
(45) Date de publication et mention de la délivrance du brevet: **14.08.2024**
(21) Numéro de dépôt: 19161434.6
(22) Date de dépôt: 07.03.2019
(51) Int. Cl.: A61B 5/08, A61B 5/097, G01N 1/22, G01N 1/40, A61B 5/083

(54) **SYSTÈME DE PRÉLÈVEMENT D'UN ÉCHANTILLON D'AIR EXPIRÉ PAR UN ÊTRE VIVANT**
SYSTEM ZUR PROBENAHME EINER VON EINEM LEBEWESEN AUSGEATMETEN LUFT
SYSTEM FOR TAKING A SAMPLE OF AIR BREATHED BY A LIVING BEING

(30) Priorité: 14.03.2018 FR 1852212
(43) Date de publication de la demande: 18.09.2019
(73) Titulaire: Commissariat à l'Énergie Atomique et aux Énergies Alternatives, 75015 Paris (FR)
(72) Inventeur: CHAPPUIS, Thomas, 38000 Grenoble (FR); CELLIER, Morgan, 34970 Lattes (FR); BOURLON, Bertrand, 38950 Saint Martin le Vinoux (FR)
(74) Mandataire: INNOV-GROUP

(56) Documents cités:
- US-A1- 2015 301 021
- US-A1- 2017 030 892
- HERBIG J ET AL: "Buffered end-tidal (BET) sampling - A novel method for real-time breath-gas analysis", JOURNAL OF BREATH RESEARCH, INSTITUTE OF PHYSICS PUBLISHING, US, vol. 2, no. 3, 1 September 2008 (2008-09-01), pages 1 - 9, XP002510726, ISSN: 1752-7155, DOI: 10.1088/1752-7155/2/3/037008
- LAWAL OLUWASOLA ET AL: "Exhaled breath analysis: areview of 'breath-taking' methods for off-line analysis", METABOLOMICS, vol. 1, no. 13, 19 August 2017 (2017-08-19), pages 110, XP055520251

## Description

### Domaine technique de l'invention

La présente invention se rapporte à un système de prélèvement d'un échantillon d'air expiré par un être vivant. L'invention concerne également un procédé de prélèvement d'un échantillon d'air expiré mis en oeuvre grâce audit dispositif, pour analyse ultérieur. L'invention concerne également un système d'analyse portable incluant ledit système de prélèvement.

### Etat de la technique

L'air expiré, terme plus exact pour désigner l'haleine, est un fluide biologique pouvant être utilisé pour le dépistage médical et le suivi d'exposition à des polluants. En effet, la composition de l'haleine change en cas de maladie, ou d'exposition à des polluants.

De manière générale, une analyse se déroule en plusieurs étapes : le prélèvement, la préconcentration et l'injection dans un système de prélèvement par thermo-désorption.

Différents systèmes ont déjà été décrits dans l'état de la technique. La demande de brevet GB2363196 décrit une solution comprenant un tube dans lequel le patient vient simplement souffler. Ce dispositif est très peu invasif pour le patient. Il prélève uniquement l'air de fin de respiration. En soufflant à travers le tube, le patient "pousse" l'air de début de respiration avec l'air de fin de respiration. Même si une partie spécifique de l'air expiré est sélectionné, cela reste peu précis. Le système est simple à utiliser et jetable. Cependant il ne permet de prélever qu'une seule expiration, ce qui limite la quantité de matière disponible. De plus, seule une partie de l'air de fin d'expiration d'une seule expiration est prélevée. L'échantillon d'air prélevé est ensuite transféré vers un dispositif de préconcentration par une action manuelle dans laquelle l'air est poussé à travers le tube grâce à un système de seringue intégré dans le tube. Ce système est compatible avec différents dispositifs de préconcentration connus.

Les demandes de brevet US2017/303822A1 et US2017/303823A1 décrivent toutes deux une solution composée d'un masque et d'un dispositif contenant un capteur de CO₂ et de pression. La solution proposée permet notamment de choisir très précisément quelle partie de l'air expiré est prélevée. Grâce aux informations fournies par les capteurs, des pompes sont commandées pour transférer directement l'air expiré vers des tubes de thermo-désorption. Cette solution permet de transférer directement l'air expiré vers le dispositif de préconcentration employé. De plus, il est possible de prélever une quantité illimitée d'air expiré selon les besoins analytiques. Cependant, comme la différence de débit lors de l'expiration (environ 10 l/min) et le débit de prélèvement du dispositif qui est au maximum de 1 l/min est importante, il s'avère que seule une partie de l'air expiré est prélevée à chaque expiration. La durée de prélèvement pendant laquelle le masque doit être porté par le patient s'avère particulièrement longue pour obtenir une quantité suffisante d'échantillon d'air expiré à analyser.

Par ailleurs, certains dispositifs de préconcentration ont déjà été décrits dans l'état de la technique. Un premier dispositif est basé sur le principe de la micro extraction sur phase solide (SPME), consistant en une fibre fonctionnalisée. La préconcentration est basée sur des équilibres thermodynamiques ce qui peut limiter le facteur de préconcentration. La quantité d"adsorbant est aussi limité ce qui limite la quantité de matière extraite.

Un autre dispositif se présente sous la forme d'un tube fonctionnant sous le principe de thermo-désorption (TD). L'échantillon passe à travers le tube contenant une poudre qui retient les molécules d'intérêt. Quand le tube est chauffé, ces molécules sont relarguées et injectées grâce à un gaz vecteur dans le système de prélèvement. Pour être couplé à différents systèmes d'analyse, un appareil d'injection complexe (nombreuses vannes, pièces chauffantes, pièces mécanique) est nécessaire. Pour chauffer ces tubes rapidement (100 °C/s) une puissance importante est également nécessaire. De plus les différences de débits pour désorber le tube (20 à 100 ml/min) et les débits des systèmes analytiques (1 ml/min) nécessitent des systèmes de vannes pour au final n'envoyer qu'une petite partie de l'échantillon dans le système analytique. Une partie de l'échantillon est ainsi perdue lors de l'injection.

Le "needle trap device" (NTD) est un mélange entre la SPME et la thermo-désorption (TD). Un tube de seringue contient une poudre adsorbante. L'échantillon d'air est pompé à travers la seringue pour être prélevé.

Un autre système d'analyse d'échantillons d'air expiré par un être vivant est décrit dans le document US 2015/301021 A1.

Le but de l'invention est de proposer un système de prélèvement d'air qui soit facile à manipuler et qui puisse réaliser une analyse fiable, sans nécessiter une durée de prélèvement trop longue.

### Exposé de l'invention

Ce but est atteint par un système de prélèvement d'un échantillon d'air expiré par un être vivant, ledit système comportant:
- Un embout d'entrée par lequel l'échantillon d'air expiré est injecté,
- Une vanne d'entrée sur laquelle est connecté l'embout d'entrée,
- Un sac de prélèvement relié audit embout d'entrée, en aval de la vanne d'entrée, afin de recueillir l'échantillon d'air expiré,
- Un dispositif de préconcentration,
- Un tuyau de transfert pour relier le sac de prélèvement au dispositif de préconcentration,
- Des moyens de transfert de l'échantillon d'air expiré prélevé dans ledit sac de prélèvement vers ledit dispositif de préconcentration à travers ledit tuyau de transfert,
- Une unité de commande configurée pour exécuter une séquence de commande comprenant une commande desdits moyens de transfert pour transférer un échantillon prélevé dans ledit sac de prélèvement vers ledit dispositif de préconcentration,

La séquence de commande étant configurée pour mettre en oeuvre les étapes suivantes :
- Surveiller l'air expiré afin de détecter la présence de l'air de fin d'expiration;
- Contrôler la vanne d'entrée pour assurer l'injection de l'air de fin d'expiration uniquement vers le sac de prélèvement ;
- Commander les moyens de transfert pour assurer un transfert de l'échantillon d'air expiré prélevé dans le sac de prélèvement vers le dispositif de préconcentration.

Selon une particularité, le dispositif de préconcentration est un microcomposant en silicium comprenant une cavité dans laquelle est placé un composant adsorbant.

Selon une autre particularité, le dispositif de préconcentration comporte une résistance chauffante et une sonde de température.

Selon une autre particularité, les moyens de transfert comportent une pompe configurée pour aspirer l'échantillon d'air expiré présent dans ledit sac de prélèvement et le transférer vers le dispositif de préconcentration.

Selon une autre particularité, le système comporte des moyens de détection de présence de l'air de fin d'expiration.

Selon une autre particularité, les moyens de détection de l'air de fin d'expiration comportent un capteur et un module de détection exécuté par l'unité de commande et configuré pour détecter la présence de l'air de fin d'expiration en fonction des données reçues dudit capteur.

Selon une autre particularité, ledit capteur est de type capteur de CO₂ ou capteur de pression.

Selon une autre particularité, la vanne d'entrée est de type trois voies, commandée par l'unité de commande et configurée pour connecter ledit embout d'entrée soit audit capteur, soit audit sac de prélèvement.

Selon une autre particularité, la séquence de commande est configurée pour mettre en oeuvre les étapes suivantes :
- Surveiller l'air expiré afin de détecter la présence de l'air de fin d'expiration ;
- Contrôler la vanne d'entrée pour assurer l'injection de l'air de fin d'expiration uniquement vers le sac de prélèvement ;
- Commander les moyens de transfert pour assurer un transfert de l'échantillon d'air expiré prélevé dans le sac de prélèvement vers le dispositif de préconcentration.

L'invention concerne également un procédé de prélèvement d'air expiré par un être vivant, ledit procédé étant mis en oeuvre à l'aide d'un système de prélèvement tel que défini ci-dessus, ledit procédé comportant les étapes suivantes :
- Surveillance de l'air expiré afin de détecter la présence de l'air de fin d'expiration ;
- Contrôle de la vanne d'entrée pour assurer l'injection de l'air de fin d'expiration uniquement vers le sac de prélèvement ;
- Commande des moyens de transfert pour assurer un transfert de l'échantillon d'air expiré prélevé dans le sac de prélèvement vers le dispositif de préconcentration.

L'invention concerne également un système d'analyse d'air expiré par un être vivant, ledit système d'analyse comportant un système de prélèvement tel que défini ci-dessus et un ensemble d'analyse connecté au dispositif de préconcentration pour recueillir des molécules piégées par ledit dispositif de préconcentration après désorption.

On comprend que l'invention concerne plus précisément l'étape de prélèvement et de concentration de l'air expiré. En effet une analyse directe d'un échantillon d'air expiré (souffler dans une machine qui analyse) ne permet souvent d'analyser que certains composés. Pour une analyse la plus exhaustive possible, un prélèvement sélectif de l'air expiré, suivi d'une étape de piégeage des molécules est indispensable. Tout l'air expiré par le patient n'est en effet pas intéressant. Le plus souvent, seul l'air "de fin de respiration" est intéressant. De plus, comme la concentration des molécules d'intérêt dans l'air expiré est faible, piéger les molécules contenues dans plusieurs expirations pour ensuite injecter tout le prélèvement d'un seul coup augmente les quantités de matières analysées et permet de détecter plus de molécules d'intérêt.

De plus, on peut noter que la puce employée dans le système de l'invention est autonome pour le chauffage et le contrôle de la température. Cela ne nécessite aucun autre équipement, ce qui n'est pas le cas pour le tube de TD, la SPME ou le NTD décrits ci-dessus.

### Brève description des figures

D'autres caractéristiques et avantages vont apparaître dans la description détaillée qui suit faite en regard des dessins annexés dans lesquels :
- La figure 1 représentent une première variante de réalisation du système de prélèvement de l'invention et la figure 2 (étapes E1 à E4) illustre son principe de fonctionnement ;
- La figure 3 représente une deuxième variante de réalisation du système de prélèvement de l'invention et la figure 4 (étapes E10 à E30) illustre son principe de fonctionnement ;
- Les figures 5A et 5B représentent de manière schématique un dispositif de préconcentration employé dans le système de prélèvement d'air expiré de l'invention ;

### Description détaillée d'au moins un mode de réalisation

Dans la suite de la description, les termes "amont" et "aval" sont à comprendre en tenant compte du sens de circulation de l'échantillon d'air expiré dans le système.

Le système de l'invention s'appuie sur un procédé discontinu, c'est-à-dire comportant une étape principale de prélèvement dans lequel un échantillon d'air expiré par l'être vivant est d'abord prélevé dans sa totalité, une étape de préconcentration pour piéger les molécules d'intérêt présentes dans l'échantillon prélevé grâce à un dispositif de préconcentration et optionnellement une étape d'analyse dans laquelle les molécules d'intérêt sont analysés, après désorption, par un ensemble d'analyse.

Le système de prélèvement comporte ainsi un dispositif de prélèvement. Le dispositif de prélèvement est avantageusement un sac de prélèvement 1 de type Tedlar (Marque déposée). Il est très peu invasif pour le patient. Le patient a juste à souffler à travers un embout (IN1, IN10) relié au sac. Il existe de nombreuses tailles de sac qui permettent de sélectionner le volume adéquat.

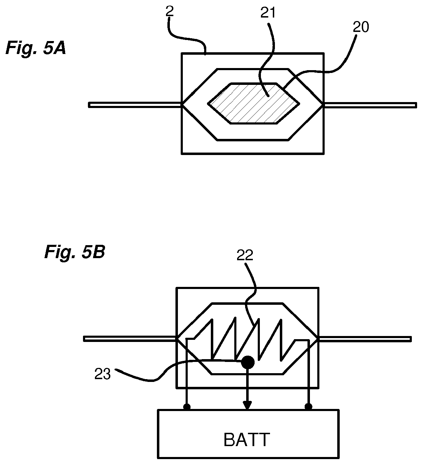

Le système de prélèvement comporte ensuite un dispositif de préconcentration 2. Ce dispositif 2 est relié directement à une connexion fluidique du sac de prélèvement 1. Le dispositif de préconcentration 2 permet d'accumuler, à l'aide d'un adsorbant, les composés à détecter présents dans l'échantillon gazeux et permet de les libérer par désorption thermique, sous l'effet d'une montée brutale de la température. Une amplification de la concentration et donc du signal pourra ainsi être obtenue. En référence aux figures 5A et 5B, un tel dispositif de préconcentration 2 est par exemple constitué d'un microcomposant ou puce en silicium et verre gravé d'une cavité 20 remplie d'au moins un adsorbant 21 (par exemple particules de Tenax - Marque déposée) et muni d'une résistance de chauffage 22 sur sa face inférieure et d'une sonde de température 23. Le dispositif de préconcentration 2 pourra se présenter sous la forme d'une cartouche amovible, à retirer du système pour être connectée sur une installation d'analyse. Des capillaires métalliques ou en silice permettent d'assurer la circulation de l'échantillon dans le dispositif. Des micro-piliers peuvent être agencés devant la sortie du dispositif pour empêcher la fuite des particules d'adsorbant utilisées.

La solution de chauffage qui permet la désorption des composés piégés est directement intégré dans la puce qui permet le prélèvement. La petite taille de la puce lui permet d'être chauffée rapidement (20°C/s) et à haute température (250 °C) avec une faible puissance car la capacité thermique du dispositif est faible. Ainsi le chauffage peut être mis en oeuvre à l'aide d'une simple batterie BATT de 12 Volts.

Le système de prélèvement comporte un circuit fluidique permettant de mettre en oeuvre le procédé de prélèvement. De manière non limitative, ce circuit fluidique comporte des tuyaux ou capillaires, notamment le tuyau de transfert T1, T10 décrit ci-dessous.

Le système de prélèvement comporte également un dispositif de transfert de l'air expiré contenu dans le sac de prélèvement vers le dispositif de préconcentration. Ce dispositif de transfert peut comporter au moins une pompe P agencée dans le circuit fluidique pour aspirer l'échantillon qui a été prélevé dans le sac de prélèvement 1 et plusieurs vannes, commandables ou non, pour permettre le transfert via le circuit fluidique.

De manière générale mais non limitative, le dispositif de transfert pourra comporter au moins une vanne d'entrée destinée à empêcher toute sortie d'air lorsque le sac de prélèvement a été rempli. Il pourra comporter en plus une solution de commande agencée sur le tuyau de transfert pour commander le transfert de l'air du sac de prélèvement vers le dispositif de préconcentration. On verra ci-dessous que, dans le premier mode de réalisation, il peut s'agir de doter le tuyau de transfert d'une section plus faible que celle du tuyau de sortie pour augmenter la résistance fluidique du tuyau. Une vanne commandée peut également être placée sur le tuyau de transfert. Dans le deuxième mode de réalisation, une vanne V20 est placée sur le tuyau de transfert pour commander le transfert d'air du sac de prélèvement 1 vers le dispositif de préconcentration 2.Le système comporte avantageusement une unité de commande UC, chargée de commander le dispositif de transfert, c'est-à-dire les vannes présentes et la pompe P, et le dispositif de préconcentration 2, en fonction notamment des données reçues par le capteur S et par la sonde de température 23 du dispositif de préconcentration 2. L'unité de commande UC est configurée pour exécuter une séquence de commande comprenant les différentes étapes nécessaires à la mise en oeuvre du prélèvement et éventuellement du transfert vers une installation d'analyse. L'unité de commande est avantageusement alimentée par la batterie BATT du système, déjà utilisé pour chauffer la résistance 22 du dispositif de préconcentration 2.

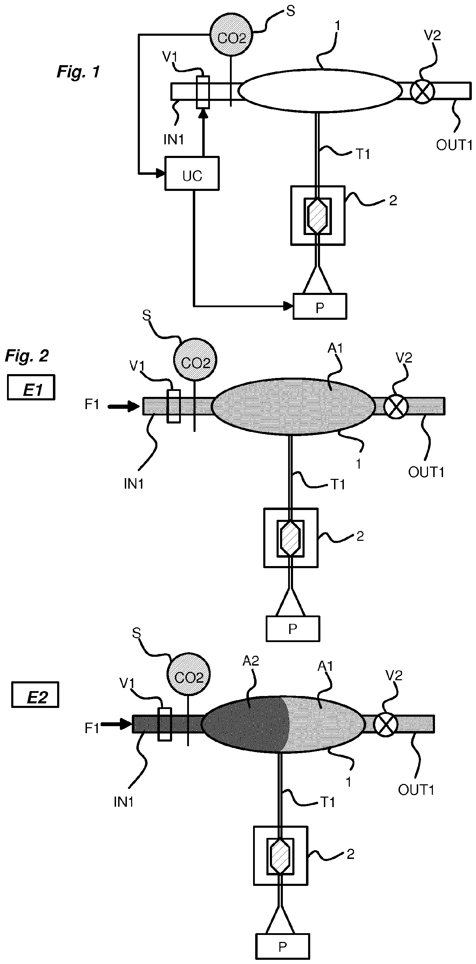

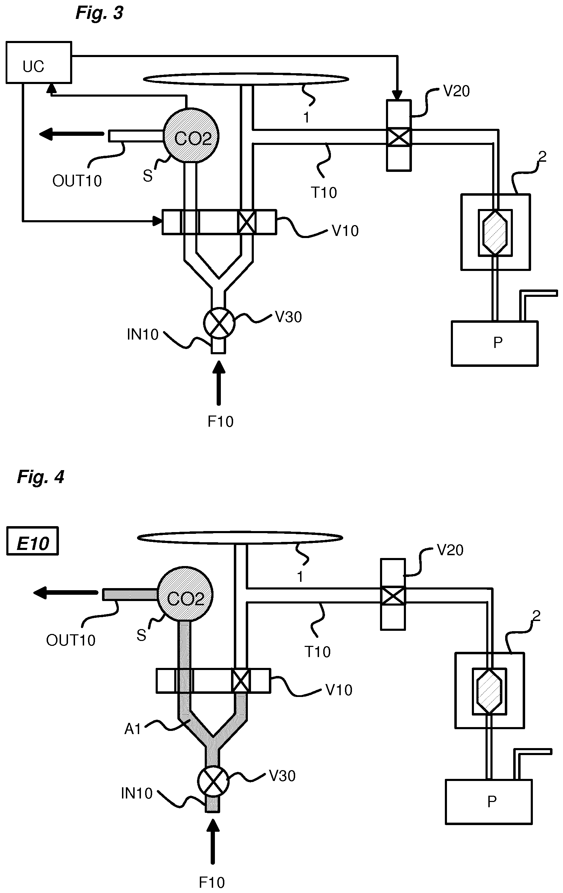

Partant des différents composants décrits ci-dessus, la figure 1 présente un premier mode de réalisation du système de prélèvement de l'invention et la figure 3 présente un deuxième mode de réalisation du système de prélèvement.

Sur la figure 1, le système comporte :
- Un embout d'entrée IN1 dans lequel le patient souffle pour introduire l'air expiré dans le système. Cet embout pourra être jetable.
- Une vanne d'entrée V1 sur laquelle est connecté ledit embout. Cette vanne V1 est ouverte lorsque le patient souffle et est fermée lorsque le patient a terminé d'expirer afin de conserver l'échantillon dans le système. La fermeture peut être contrôlée manuellement ou de manière automatique en employant un capteur S de pression ou de CO₂. Un débitmètre peut également être employé pour mesurer la quantité d'air expiré, notamment la quantité d'air de fin d'expiration.
- Un sac de prélèvement 1 conforme à celui décrit ci-dessus, connecté audit embout d'entrée, en aval de ladite vanne d'entrée V1.
- Un tuyau de sortie OUT1 connecté au sac de prélèvement 1, en aval de celui-ci par rapport au tuyau d'entrée IN1.
- Une vanne de sortie V2 agencée sur le tuyau de sortie OUT1, de type anti-retour.
- Un tuyau de transfert T1 connecté au sac de prélèvement 1, en parallèle du tuyau de sortie OUT1. Le tuyau de transfert peut présenter une section transversale plus faible que celle du tuyau de sortie, de manière à disposer d'une résistance fluidique plus élevée que celle du tuyau de sortie. Une vanne commandable peut également être placée sur le tuyau de transfert T1 (comme dans le deuxième mode de réalisation).
- Un dispositif de préconcentration 2 connecté audit tuyau de transfert T1 pour recevoir l'échantillon prélevé lors du transfert après prélèvement.
- Une pompe P connectée en aval du dispositif de préconcentration 2 et commandée par l'unité de commande UC pour aspirer l'échantillon prélevé en dehors du sac de prélèvement 1 et l'amener dans le dispositif de préconcentration 2 lors du transfert.
- Une unité de commande UC recevant en entrée les données du capteur S de pression ou de CO₂ si un tel capteur est présent, les données de température en provenance de la sonde 23 du dispositif de préconcentration 2 et qui envoie des commandes à destination de la vanne d'entrée V1 en fonction des données reçues du capteur S de pression ou du capteur de CO₂ et à destination de la pompe P pour commander l'aspiration de l'échantillon prélevé dans le sac de prélèvement 1 vers le dispositif de préconcentration 2. L'unité de commande UC met en oeuvre une séquence de commande adaptée au principe de fonctionnement du système. La séquence de commande peut comporter des instructions logicielles successives exécutées par l'unité de commande en vue de réaliser les différentes étapes du procédé de prélèvement.

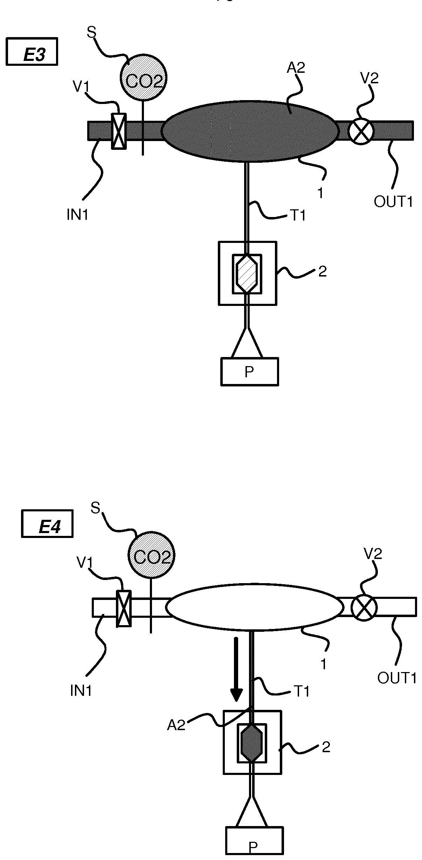

Les différentes étapes de la séquence de commande du système de la figure 1 sont détaillées ci-dessous en liaison avec la figure 2 :

### - Etape E1

Le patient souffle dans le système par l'embout d'entrée IN1 afin de faire pénétrer l'air expiré (flèche F1) dans le système. Comme décrit ci-dessus, la partie pertinente de l'air expiré est présente en fin d'expiration. L'air de début d'expiration A1 est donc rejeté par le tuyau de sortie OUT1 après être passé par le sac de prélèvement 1, la vanne de sortie V2 de type anti-retour étant ouverte par la pression exercée lors de l'expiration.

### - Etape E2

L'air de fin d'expiration A2 pénètre dans le sac de prélèvement 1. L'air de début d'expiration A1 est poussé vers l'extérieur.

### - Etape E3

Grâce à l'emploi d'un capteur S de pression ou de CO2 et à un module de détection exécuté par l'unité de commande UC, il est possible de savoir que l'air de fin d'expiration A2 est désormais présent dans le sac de prélèvement S1. L'unité de commande UC contrôle alors la fermeture de la vanne d'entrée V1. Une commande manuelle peut également être mise en oeuvre par le patient. Le sac de prélèvement 1 est rempli d'air de fin d'expiration A2 et ne contient donc qu'un échantillon pertinent pour analyse.

### - Etape E4

L'unité de commande UC contrôle la pompe P pour aspirer l'échantillon d'air prélevé dans le sac de prélèvement 1 vers le dispositif de préconcentration 2, via le tuyau de transfert T1. Grâce à son adsorbant, le dispositif de préconcentration 2 piège les molécules d'intérêt. Une fois la préconcentration terminée, l'unité de commande UC peut commander le chauffage du dispositif de préconcentration 2, grâce à sa résistance 22, afin de désorber les molécules et les transférer sous forme gazeuse vers un ensemble d'analyse adapté. L'ensemble d'analyse peut être intégré au système.

Sur la figure 3, selon le deuxième mode de réalisation, le système comporte :
- Un embout d'entrée IN10 dans lequel le patient souffle pour introduire l'air expiré dans le système. Cet embout pourra être jetable.
- Une vanne V30 de type anti-retour sur laquelle est connecté ledit embout. Cette vanne est ouverte lorsque le patient souffle et est fermée lorsque le patient a terminé d'expirer.

- Une vanne d'entrée V10 trois voies sur laquelle est connecté l'embout d'entrée.
- Un tuyau de sortie OUT10 sur lequel est placé un capteur S de CO₂ connecté à l'embout d'entrée IN10 via la vanne d'entrée V10 trois voies.
- Un sac de prélèvement 1 conforme à celui décrit ci-dessus, connecté audit embout d'entrée, via la vanne d'entrée V10 trois voies. La vanne d'entrée V10 trois voies permet de connecter l'embout d'entrée IN10, soit au capteur S de CO2 et au tuyau de sortie OUT10, soit au sac de prélèvement 1, ou peut être complètement fermée. La capacité du sac de prélèvement 1 pourra être choisie suffisante pour prélever l'air d'une seule expiration ou de plusieurs expirations.
- Un tuyau de transfert T10 connecté au sac de prélèvement 1.
- Une vanne de transfert V20 deux voies commandée, placée sur le tuyau de transfert T10.
- Un dispositif de préconcentration 2 connecté audit tuyau de transfert pour recevoir l'échantillon prélevé lors du transfert après prélèvement.
- Une pompe P connectée en aval du dispositif de préconcentration et commandée pour aspirer l'échantillon prélevé en dehors du sac de prélèvement et l'amener dans le dispositif de préconcentration lors du transfert.
- Une unité de commande UC recevant en entrée les données du capteur S de pression ou de CO₂ si un tel capteur est présent, les données de température en provenance de la sonde 23 du dispositif de préconcentration et qui envoie des commandes à destination de la vanne d'entrée V10 si celle-ci est automatique en fonction des données reçues du capteur S de pression ou du capteur de CO₂, à destination de la vanne de transfert V20 et de la pompe P pour commander l'aspiration de l'échantillon prélevé dans le sac de prélèvement 1 vers le dispositif de préconcentration 2. L'unité de commande UC met en oeuvre une séquence de commande adaptée au principe de fonctionnement du système. La séquence de commande peut comporter des instructions logicielles successives exécutées par l'unité de commande en vue de réaliser les différentes étapes du procédé de prélèvement.

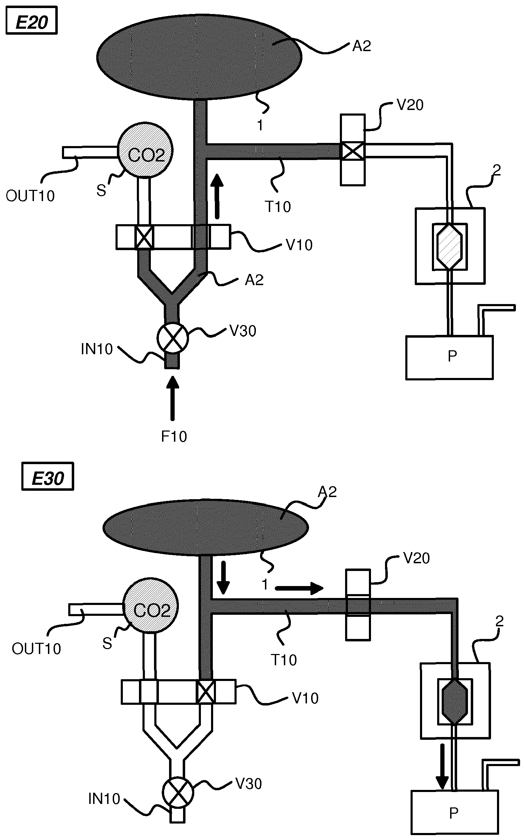

Les différentes étapes de la séquence de commande du système de la figure 3 sont détaillées ci-dessous en liaison avec la figure 4 :

### - Etape E10

Le patient souffle dans le système par l'embout d'entrée IN10 afin de faire pénétrer l'air expiré dans le système (flèche F10). Comme décrit ci-dessus, la partie pertinente de l'air expiré est présente en fin d'expiration. La vanne d'entrée V10 est commutée dans une position de connexion de l'embout d'entrée IN10 vers le tuyau de sortie OUT10, à travers le capteur de CO₂ et reste dans cet état tant que la présence de l'air de fin d'expiration A2 n'a pas été détectée. L'air de début d'expiration A1 est donc rejeté par le tuyau de sortie OUT10. Les données recueillies par le capteur de CO₂ permettent à l'unité de commande de détecter à quel moment l'air de fin d'expiration A2 commence à pénétrer dans le système.

### - Etape E20

Lorsque la présence de l'air de fin d'expiration A2 est détectée, la vanne d'entrée V10 est commutée par l'unité de commande UC de manière à dévier l'air expiré vers le sac de prélèvement 1. L'air expiré pénètre dans le sac de prélèvement 1. La vanne de transfert reste fermée, ne permettant pas le transfert vers le dispositif de préconcentration.

### - Etape E30

L'unité de commande UC contrôle ensuite la fermeture de la vanne d'entrée V10 afin de conserver l'échantillon d'air expiré dans le sac de prélèvement 1 et d'éviter qu'il ne s'échappe. L'unité de commande UC contrôle l'ouverture de la vanne de transfert V20. L'unité de commande UC contrôle la pompe P pour aspirer l'échantillon d'air expiré prélevé dans le sac de prélèvement 1 vers le dispositif de préconcentration, à travers le tuyau de transfert T20 et la vanne de transfert V20 qui est alors ouverte. Grâce à son adsorbant, le dispositif de préconcentration 2 piège les molécules d'intérêt. Une fois la préconcentration terminée, l'unité de commande UC peut commander le chauffage du dispositif de préconcentration 2, grâce à la résistance 22, afin de désorber les molécules et de les transférer sous forme gazeuse vers un ensemble d'analyse adapté. L'ensemble d'analyse peut être intégré au système.

Dans ses deux réalisations, le système est avantageusement assemblé dans un unique boîtier plastique intégrant les différents composants, notamment le dispositif de préconcentration 2, la pompe P, les vannes, le circuit fluidique et l'unité de commande UC. Le sac de prélèvement 1 peut être facilement connecté sur ledit boîtier et l'embout d'entrée IN1, IN10 est amovible sur ledit boîtier. L'ensemble d'analyse peut également être intégré au boîtier du système ou déporté par rapport à celui-ci.

Le système de l'invention permet d'atteindre les mêmes limites de détection que les solutions existantes en prélevant une seule expiration. Cela est permis grâce à deux caractéristiques du dispositif :
- Tout l'air expiré est prélevé lors de chaque expiration et,
- Toute la quantité de matière prélevée sur le dispositif de préconcentration 2 est transférée dans l'ensemble d'analyse.

Un autre intérêt du système est de pouvoir être intégré dans un système portable. Comme la puce de prélèvement est "petite" et que le volume d'échantillon à prélever est plus faible, la taille de la pompe P est réduite et la consommation électrique est moindre ce qui peut permettre de travailler sur batterie (BATT).

Les solutions de l'invention permettent de transférer l'intégralité de l'air expiré prélevé vers le dispositif de préconcentration, contrairement aux solutions antérieures déjà évoquées où au moins 90% de l'échantillon est perdu lors du transfert. Dans l'état de la technique, en combinant les étapes de prélèvement et d'injection, au moins 98% de l'air expiré n'est pas injecté dans le système et le prélèvement dure souvent au moins 15 minutes, alors qu'il peut être réalisé en moins d'une dizaine de seconde, ce qui correspond à une ou deux expirations, grâce à la solution de l'invention.

Le dispositif de préconcentration 2 sous la forme de microcomposant a notamment été validé dans la détection de marqueurs de tabagisme dans l'air expiré, chez des fumeurs et des non-fumeurs. Les marqueurs suivis étaient le toluène, le benzène et le 2, 5, dimethylfuran. Les marqueurs sont beaucoup plus concentrés dans l'air expiré des fumeurs que dans l'air expiré des non-fumeurs. La puce seule a été utilisée en prélevant un échantillon prélevé grâce à un sac de prélèvement de type Tedlar.

Bien entendu, certaines adaptations pourront être envisagées. Le capteur de CO₂ pourra notamment être remplacé par une simple temporisation déclenchée au début de l'expiration, l'unité de commande activant le prélèvement de l'air de fin d'expiration dans le sac de prélèvement après une certaine durée, considérée comme standard.

L'invention est définie par les revendications ci-jointes.

## Revendications

1. Système de prélèvement d'un échantillon d'air expiré par un être vivant, ledit système comportant:
- Un embout d'entrée (IN1, IN10) par lequel l'échantillon d'air expiré est injecté,
- Une vanne d'entrée (V1, V10) sur laquelle est connecté l'embout d'entrée (IN1, IN10),
- Un sac de prélèvement (1) relié audit embout d'entrée (IN1, IN10), en aval de la vanne d'entrée, afin de recueillir l'échantillon d'air expiré,
- Un dispositif de préconcentration (2),
- Un tuyau de transfert (T1, T10) pour relier le sac de prélèvement (1) au dispositif de préconcentration (2),
- Des moyens de transfert de l'échantillon d'air expiré prélevé dans ledit sac de prélèvement (1) vers ledit dispositif de préconcentration (1) à travers ledit tuyau de transfert,
- Une unité de commande (UC) configurée pour exécuter une séquence de commande comprenant une commande desdits moyens de transfert pour transférer un échantillon prélevé dans ledit sac de prélèvement (1) vers ledit dispositif de préconcentration (2),
La séquence de commande étant configurée pour mettre en oeuvre les étapes suivantes :
- Surveiller l'air expiré afin de détecter la présence de l'air de fin d'expiration (A2) ;
- Contrôler la vanne d'entrée (V1, V10) pour assurer l'injection de l'air de fin d'expiration (A2) uniquement vers le sac de prélèvement (1) ;
- Commander les moyens de transfert pour assurer un transfert de l'échantillon d'air expiré prélevé dans le sac de prélèvement (1) vers le dispositif de préconcentration (2).

2. Système selon la revendication 1, **caractérisé en ce que** le dispositif de préconcentration (2) est un microcomposant en silicium comprenant une cavité (20) dans laquelle est placé un composant adsorbant (21).

3. Système selon la revendication 2, **caractérisé en ce que** le dispositif de préconcentration (2) comporte une résistance chauffante (22) et une sonde de température (23).

4. Systèmeselon l'une des revendications 1 à 3, **caractérisé en ce que** les moyens de transfert comportent une pompe (P) configurée pour aspirer l'échantillon d'air expiré présent dans ledit sac de prélèvement (1) et le transférer vers le dispositif de préconcentration (2).

5. Système selon l'une des revendications 1 à 4, **caractérisé en ce qu'**il comporte des moyens de détection de présence de l'air de fin d'expiration (A2).

6. Système selon la revendication 5, **caractérisé en ce que** les moyens de détection de l'air de fin d'expiration (A2) comportent un capteur (S) et un module de détection exécuté par l'unité de commande (UC) et configuré pour détecter la présence de l'air de fin d'expiration (A2) en fonction des données reçues dudit capteur (S).

7. Système selon la revendication 6, **caractérisé en ce que** ledit capteur (S) est de type capteur de CO₂ ou capteur de pression.

8. Système selon la revendication 6 ou 7, **caractérisé en ce que** la vanne d'entrée (V10) est de type trois voies, commandée par l'unité de commande (UC) et configurée pour connecter ledit embout d'entrée (IN10) soit audit capteur (S), soit audit sac de prélèvement (1).

9. Procédé de prélèvement d'air expiré par un être vivant, ledit procédé étant mis en oeuvre à l'aide d'un système de prélèvement tel que défini dans l'une des revendications 5 à 8, et en ce qu'il comporte les étapes suivantes :
- Surveillance de l'air expiré afin de détecter la présence de l'air de fin d'expiration (A2) ;
- Contrôle de la vanne d'entrée (V1, V10) pour assurer l'injection de l'air de fin d'expiration (A2) uniquement vers le sac de prélèvement (1) ;
- Commande des moyens de transfert pour assurer un transfert de l'échantillon d'air expiré prélevé dans le sac de prélèvement (1) vers le dispositif de préconcentration (2).

10. Système d'analyse d'air expiré par un être vivant, **caractérisé en ce qu'**il comporte un système de prélèvement tel que défini dans l'une des revendications 1 à 8 et un ensemble d'analyse connecté au dispositif de préconcentration (2) pour recueillir des molécules piégées par ledit dispositif de préconcentration (2) après désorption.

## Patentansprüche

1. System zur Entnahme einer Probe von Luft, die von einem Lebewesen ausgeatmet wurde, wobei das System Folgendes aufweist:
- ein Eingangsstück (IN1, IN10), durch welches die Probe ausgeatmeter Luft eingeleitet wird,
- ein Eingangsventil (V1, V10), an welches das Eingangsstück (IN1, IN10) angeschlossen wird,
- einen Entnahmebeutel (1), der stromabwärts des Eingangsventils mit dem Eingangsstück (IN1, IN10) verbunden ist, um die Probe ausgeatmeter Luft aufzunehmen,
- eine Vorkonzentrationsvorrichtung (2),
- einen Überführungsschlauch (T1, T10), um den Entnahmebeutel (1) mit der Vorkonzentrationsvorrichtung (2) zu verbinden,
- Mittel zum Überführen der Probe ausgeatmeter Luft, die derart entnommen wurde, dass sie sich im Entnahmebeutel (1) befindet, durch den Überführungsschlauch in Richtung der Vorkonzentrationsvorrichtung (1),
- eine Steuerungseinheit (UC), die dafür ausgelegt ist, eine Steuerungssequenz auszuführen, die eine Steuerung der Überführungsmittel umfasst, um eine Probe, die derart entnommen wurde, dass sie sich im Entnahmebeutel (1) befindet, in Richtung der Vorkonzentrationsvorrichtung (2) zu überführen,
wobei die Steuerungssequenz dafür ausgelegt ist, die folgenden Schritte durchzuführen:
- Überwachen der ausgeatmeten Luft, um das Vorhandensein der Ausatmungsendluft (A2) nachzuweisen;
- Bedienen des Eingangsventils (V1, V10), derart, das sichergestellt wird, dass ausschließlich die Ausatmungsendluft (A2) in Richtung des Entnahmebeutels (1) geleitet wird;
- Steuern der Überführungsmittel, derart, dass sichergestellt wird, dass die Probe ausgeatmeter Luft, die derart entnommen wurde, dass sie sich im Entnahmebeutel (1) befindet, in Richtung der Vorkonzentrationsvorrichtung (2) überführt wird.

2. System nach Anspruch 1, **dadurch gekennzeichnet, dass** es sich bei der Vorkonzentrationsvorrichtung (2) um ein Mikrobauteil aus Silicium handelt, das einen Hohlraum (20) umfasst, in welchem ein adsorbierender Bestandteil (21) angeordnet ist.

3. System nach Anspruch 2, **dadurch gekennzeichnet, dass** die Vorkonzentrationsvorrichtung (2) einen Heizwiderstand (22) und eine Temperatursonde (23) umfasst.

4. System nach einem der Ansprüche 1 bis 3, **dadurch gekennzeichnet, dass** die Überführungsmittel eine Pumpe (P) aufweisen, die dafür ausgelegt ist, die Probe ausgeatmeter Luft, welche sich in dem Entnahmebeutel (1) befindet, anzusaugen und in Richtung der Vorkonzentrationsvorrichtung (2) zu überführen.

5. System nach einem der Ansprüche 1 bis 4, **dadurch gekennzeichnet, dass** es Mittel zum Nachweisen des Vorhandenseins der Ausatmungsendluft (A2) aufweist.

6. System nach Anspruch 5, **dadurch gekennzeichnet, dass** die Mittel zum Nachweisen der Ausatmungsendluft (A2) einen Sensor (S) sowie ein Nachweismodul aufweisen, dessen Ausführung durch die Steuerungseinheit (UC) erfolgt, wobei es dafür ausgelegt ist, das Vorhandensein der Ausatmungsendluft (A2) in Abhängigkeit von den Daten nachzuweisen, welche vom Sensor (S) empfangen werden.

7. System nach Anspruch 6, **dadurch gekennzeichnet, dass** der Sensor (S) vom Typ CO₂-Sensor oder Drucksensor ist.

8. System nach Anspruch 6 oder 7, **dadurch gekennzeichnet, dass** das Eingangsventil (V10) vom Dreiwegetyp ist, wobei es von der Steuerungseinheit (UC) gesteuert wird und dafür ausgelegt ist, das Eingangsstück (IN10) entweder an den Sensor (S) oder an den Entnahmebeutel (1) anzuschließen.

9. Verfahren zur Entnahme von Luft, die von einem Lebewesen ausgeatmet wird, wobei das Verfahren mit Hilfe eine Entnahmesystems gemäß der Begriffsbestimmung in einem der Ansprüche 5 bis 8 durchgeführt wird, und dadurch, dass es die folgenden Schritte aufweist:
- Überwachen der ausgeatmeten Luft, um das Vorhandensein der Ausatmungsendluft (A2) nachzuweisen;
- Bedienen des Eingangsventils (V1, V10), derart, das sichergestellt wird, dass ausschließlich die Ausatmungsendluft (A2) in Richtung des Entnahmebeutels (1) geleitet wird;
- Steuern der Überführungsmittel, derart, dass sichergestellt wird, dass die Probe ausgeatmeter Luft, in den Entnahmebeutel (1) gelangt ist, in Richtung der Vorkonzentrationsvorrichtung (2) überführt wird.

10. System zur Untersuchung von Luft, die von einem Lebewesen ausgeatmet wird, **dadurch gekennzeichnet, dass** es ein Entnahmesystem gemäß einem der Ansprüche 1 bis 8 sowie eine Untersuchungsanordnung aufweist, welche an die Vorkonzentrationsvorrichtung (2) angeschlossen ist, um Moleküle, die von der Vorkonzentrationsvorrichtung (2) aufgefangen wurden, nach deren Desorption in aufzunehmen.

## Claims

1. System for taking a sample of air exhaled by a living being, said system comprising:
- an inlet nozzle (IN1, IN10) through which the sample of exhaled air is injected,
- an inlet valve (V1, V10) to which the inlet nozzle (IN1, IN10) is connected,
- a sampling bag (1) connected to said inlet nozzle (IN1, IN10), downstream of the inlet valve, in order to collect the sample of exhaled air,
- a preconcentration device (2),
- a transfer pipe (T1, T10) for connecting the sampling bag (1) to the preconcentration device (2),
- means for transferring the sample of exhaled air from said sampling bag (1) to said preconcentration device (1) through said transfer pipe,
- a control unit (UC) configured to execute a control sequence comprising a control of said transfer means to transfer a sample from said sampling bag (1) to said preconcentration device (2),
the control sequence being configured to implement the following steps:
- monitoring of the exhaled air in order to detect the presence of end-expiratory air (A2);
- adjustment of the inlet valve (V1, V10) to ensure the injection of end-expiratory air (A2) only to the sampling bag (1);
- control of the transfer means to ensure a transfer of the sample of exhaled air from the sampling bag (1) to the preconcentration device (2).

2. System according to Claim 1, **characterized in that** the preconcentration device (2) is a silicon microcomponent comprising a cavity (20) in which an adsorbent component (21) is placed.

3. System according to Claim 2, **characterized in that** the preconcentration device (2) comprises a heating resistor (22) and a temperature probe (23).

4. System according to any one of Claims 1 to 3, **characterized in that** the transfer means comprise a pump (P) configured to aspirate the sample of exhaled air present in said sampling bag (1) and transfer it to the preconcentration device (2).

5. System according to any one of Claims 1 to 4, **characterized in that** it comprises means for detecting the presence of end-expiratory air (A2).

6. System according to Claim 5, **characterized in that** the means for detecting end-expiratory air (A2) comprise a sensor (S) and a detection module which is executed by the control unit (UC) and configured to detect the presence of end-expiratory air (A2) as a function of the data received from said sensor (S).

7. System according to Claim 6, **characterized in that** said sensor (S) is of the CO₂ sensor or pressure sensor type.

8. System according to Claim 6 or 7, **characterized in that** the inlet valve (V10) is of the three-way type, controlled by the control unit (UC) and configured to connect said inlet nozzle (IN10) either to said sensor (S) or to said sampling bag (1).

9. Method for taking a sample of air exhaled by a living being, said method being carried out using a sampling system as defined in one of Claims 5 to 8 and comprising the following steps:
- monitoring of the exhaled air in order to detect the presence of end-expiratory air (A2);
- adjustment of the inlet valve (V1, V10) to ensure the injection of end-expiratory air (A2) only to the sampling bag (1);
- control of the transfer means to ensure a transfer of the sample of exhaled air from the sampling bag (1) to the preconcentration device (2).

10. System for analysing air exhaled by a living being, **characterized in that** it comprises a sampling system as defined in one of Claims 1 to 8 and an analysis assembly connected to the preconcentration device (2) in order to collect molecules trapped by said preconcentration device (2) after desorption.
